(19) Europäisches Patentamt
European Patent Office
Office européen des brevets

(11) **EP 4 249 908 A1**

(12) **EUROPEAN PATENT APPLICATION**

(43) Date of publication:
**27.09.2023 Bulletin 2023/39**

(21) Application number: **22163437.1**

(22) Date of filing: **22.03.2022**

(51) International Patent Classification (IPC):
**G01N 33/00** (2006.01)    **G01N 27/02** (2006.01)

(52) Cooperative Patent Classification (CPC):
**G01N 33/004; G01N 27/126**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Max-Planck-Gesellschaft zur Förderung der Wissenschaften e.V.**
**80539 München (DE)**

(72) Inventors:
• **STRAUSS, Volker**
**14476 Potsdam (DE)**
• **WANG, Huize**
**13353 Berlin (DE)**

(74) Representative: **Deblon, Jörg-Stephan**
**Patentanwaltskanzlei Dr. Deblon**
**Alte Honrather Strasse 22**
**53797 Lohmar (DE)**

(54) **NOVEL CARBON DIOXIDE SENSOR MATERIALS AND THEIR MANUFACTURE**

(57)    The present invention relates to novel carbon dioxide sensor compositions based on nitrogen doped carbon and their manufacture as well as to carbon dioxide sensor materials and carbon dioxide sensor devices comprising such carbon dioxide sensor compositions and carbon dioxide sensor materials.

**Fig. 4**

LP$_{O_2}$-Ade380$_{90}$/Glu300$_{10}$      50 µm

EP 4 249 908 A1

## Description

## Field of the Invention

The present invention relates to novel carbon dioxide sensor compositions based on nitrogen doped carbon and their manufacture as well as to carbon dioxide sensor materials and carbon dioxide sensor devices comprising such carbon dioxide sensor compositions and carbon dioxide sensor materials.

## Background

Close monitoring of carbon dioxide levels in air has gained significant attention in particular over the past two decades. The application areas are manifold. Detecting carbon dioxide emissions in industrial and work environments is important to ensure air quality and to prevent potential health risks such as acidosis. Other examples relate to the control of carbon dioxide levels in greenhouse plantations to optimize plant growth or to control a protective carbon dioxide atmosphere in flexible food packagings.

Owing to their high sensitivity, low-cost, and simplicity metal oxide-based resistive gas sensors are the state-of-the-art devices. However, metal oxide gas sensors generally exhibit some disadvantages such as high working temperatures and low intrinsic flexibility.

[1] WO 2020/214814 A discloses carbon dioxide sensors based on metal-organic frameworks which properly work at different levels of humidity.

Other commercial state-of-the-art carbon dioxide sensors are based on spectroscopic devices.

A promising alternative to metal oxide gas sensors are carbon nanomaterials (CNMs), such as carbon nanotubes, that are selectively sensitive to carbon dioxide already at room temperature. Typically, to achieve selectivity, CNMs are fuctionalized or used in hybrid materials further comprising metals or metal oxides on their surface to provide specific active sites or to tune the electronic properties.

However, such materials require sophisticated fabrication processes and their costly manufacturing is still prohibitive for commercial applications.

Another, more cost-effective alternative are nitrogen-doped carbons (NCs) or carbon nitrides (CN) as they intrinsically provide selective binding sites for carbon dioxide. NC's and CN's obtained from direct pyrolysis are a class of materials which have been widely studied in recent years for selective carbon dioxide capture or conversion.

In particular, polypyrrole or imidazole-based NC's have demonstrated a remarkable selectivity for carbon dioxide. Besides that these materials show high environmental stability and good electrical conductivity.

The aforementioned materials are disclosed in [2] S. Naidu Talapaneni, J. Hoon Lee, S. Hyun Je, O. Buyukcakir, T. Kwon, K. Polychronopoulou, J. Wook Choi, A. Coskun, S. N. Talapaneni, J. H. Lee, S. H. Je, O. Buyukcakir, T. Kwon, J. W. Choi, A. Coskun, K. Polychronopoulou, "Chemical Blowing Approach for Ultramicroporous Carbon Nitride Frameworks and Their Applications in Gas and Energy Storage" 2016, DOI 10.1002/adfm.201604658; [3] D. Yu, J. Hu, L. Zhou, J. Li, J. Tang, C. Peng, H. Liu, "Nitrogen-Doped Coal Tar Pitch Based Microporous Carbons with Superior CO2 Capture Performance" 2018, DOI 10.1021/acs.energyfuels.8b00125; [4] D. Wu, Z. Li, M. Zhong, T. Kowalewski, K. Matyjaszewski, "Templated Synthesis of Nitrogen-Enriched Nanoporous Carbon Materials from Porogenic Organic Precursors Prepared by ATRP**" , DOI 10.1002/anie.201309836; [5] J. Wei, D. Zhou, Z. Sun, Y. Deng, Y. Xia, D. Zhao, "A Controllable Synthesis of Rich Nitrogen-Doped Ordered Mesoporous Carbon for CO2 Capture and Supercapacitors" 2013, DOI 10.1002/adfm.201202764; [6] J. Gong, M. Antonietti, J. Yuan, "Poly(Ionic Liquid)-Derived Carbon with Site-Specific N-Doping and Biphasic Heterojunction for Enhanced CO2 Capture and Sensing" Angew. Chemie 2017, 129, 7665-7671; [7] W. Ju, A. Bagger, G.-P. Hao, A. S. Varela, I. Sinev, V. Bon, B. Roldan Cuenya, S. Kaskel, J. Rossmeisl, P. Strasser, "Understanding activity and selectivity of metal-nitrogen-doped carbon catalysts for electrochemical reduction of CO2" Nat. Commun. 2017, 8, 944; [8] M. Sevilla, P. Valle-Vigón, A. B. Fuertes, "N-Doped Polypyrrole-Based Porous Carbons for CO2 Capture" Adv. Funct. Mater. 2011, 21, 2781-2787; [9] V. Chandra, S. U. Yu, S. H. Kim, Y. S. Yoon, D. Y. Kim, A. H. Kwon, M. Meyyappan, K. S. Kim, "Highly selective CO2 capture on N-doped carbon produced by chemical activation of polypyrrole functionalized graphene sheets" Chem. Commun. 2012, 48, 735-737; [10] S. R. Venna, M. A. Carreon, "Highly Permeable Zeolite Imidazolate Framework-8 Membranes for CO2 / CH4 Separation" J. Am. Chem. Soc. 2010, 132, 76-78.

Hierarchical NC's as carbon dioxide adsorbent with high carbon dioxide selectivity from rationally designed polypyrrole precursors are disclosed in [11] John W. F. To et al., J. Am. Chem. Soc. 2016, 138, 1001-1009.

Sensors for oxygen detection made from graphitic carbon nitride and reduced graphene oxides are disclosed in [12] US 2019/0003998.

Further efforts have been undertaken to improve the adsorption of carbon dioxide in porous carbon materials by increasing the nitrogen content, introducing basic functional groups or enhancing the surface polarity (see [13] Y. Zhao, X. Liu, Y. Han, "Microporous carbonaceous adsorbents for CO2 separation via selective adsorption" RSC Adv. 2015, 5, 30310-30330; [14] Y. Zhao, X. Liu, K. X. Yao, L. Zhao, Y. Han, "Superior capture of CO2 achieved by introducing extra-framework cations into N-doped microporous carbon" Chem. Mater. 2012, 24, 4725-4734 and [15] M. Oschatz, M. Antonietti, "A search for selectivity to enable CO2 capture with porous adsorbents" Energy Environ. Sci. 2018, 11, 57-70).

In general, laser-processing has become a broadly applied technique for the fabrication of mechanical and chemical sensors with excellent electric and thermal conductivities, high surface areas, and good flexibility.

Common laser-induced carbon materials are for example obtained by laser treatment of graphene oxide, polyimides, or lignins, see [16] R. B. K. Maher F. El-Kady, Veronica Strong, Sergey Dubin, "Laser Scribing of High-Performance and Flexible Graphene-Based Electrochemical Capacitors", Science, 2012, 335, 1326-1330; [17] J. Lin, Z. Peng, Y. Liu, F. Ruiz-Zepeda, R. Ye, E. L. G. Samuel, M. J. Yacaman, B. I. Yakobson, J. M. Tour, "Laser-induced porous graphene films from commercial polymers" Nat. Commun. 2014, 5, 5-12 and [18] R. Ye, Y. Chyan, J. Zhang, Y. Li, X. Han, C. Kittrell, J. M. Tour, "Laser-Induced Graphene Formation on Wood" Adv. Mater. 2017, 29, 1702211.

Further advanced approaches are disclosed in [19] V. Strauss, S. Delacroix, A. Zieleniewska, A. J. Ferguson, J. L. Blackburn, S. Ronneberger, F. F. Loeffler, "Using carbon laser patterning to produce flexible, metal-free humidity sensors"; ACS Appl. Electron. Mater. 2020, 2, 4146-4154; [20] H. Wang, S. Delacroix, A. Zieleniewska, J. Hou, N. V. Tarakina, D. Cruz, I. Lauermann, A. J. Ferguson, J. L. Blackburn, V. Strauss, "In Situ Synthesis of Molybdenum Carbide Nanoparticles Incorporated into Laser-Patterned Nitrogen-Doped Carbon for Room Temperature VOC Sensing" Adv. Funct. Mater. 2021, 2104061; [21] H. Wang, S. Delacroix, O. Osswald, M. Anderson, T. Heil, E. Lepre, N. Lopez-Salas, R. B. Kaner, B. Smarsly, V. Strauss, "Laser-carbonization: Peering into the formation of micro-thermally produced (N-doped)carbons" Carbon N. Y. 2021, 176, 500-510, [22] S. Delacroix, H. Wang, T. Heil, V. Strauss, "Laser-Induced Carbonization of Natural Organic Precursors for Flexible Electronics" Adv. Electron. Mater. 2020, 6, 2000463 and [23] M. Hepp, H. Wang, K. Derr, S. Delacroix, S. Ronneberger, F. Loeffler, B. Butz, V. Strauss, "Trained Laser-Patterned Carbon for High-Performance Mechanical Sensors" npj Flex. Electron. 2021, DOI: 10.1038/s41528-022-00136-0.

A general overview on "Carbon nanomaterial hybrids via laser writing for high-performance non-enzymatic electrochemical sensors: a critical review" was published in [24] Marcel Simsek, Nongnoot Wongkaew, Analytical and Bioanalytical Chemistry (2021) 413:6079-6099.

Despite the efforts taken and the recent progress observed the materials known so far are still improvable, in particular with respect to their sensing properties and their manufacturing process.

As a consequence there was still a need for improved carbon dioxide sensor materials with high mechanical flexibility, good conductivities, high selectivities and a rapid response which can be used at ambient temperature and an efficient process for their manufacture.

## Summary of the Invention

According to one aspect of the invention, there is now provided a carbon dioxide sensor composition comprising at least

1) a first layer comprising a nitrogen-doped carbon material having a

a) carbon content of 75 to 95 wt-%, preferably of 75 to 90 wt.-% and

b) a nitrogen content of 2 to 15 wt.-%, preferably 5 to 15 wt.-% and

c) an oxygen content of 3 to 12 wt.-%, preferably 5 to 10 wt.-% as measured by Energy-Dispersive X-ray spectroscopy (EDX) whereby the content of carbon, nitrogen and oxygen makes up 98 % to 100 % of the total mass of the first layer and

2) a second layer comprising a nitrogen-doped carbon material having a

a) carbon content of 45 to 74 wt-%, preferably of 50 to 74 wt.-% and
b) a nitrogen content of 22 to 45 wt.-%, preferably 23 to 42 wt.-% and
c) an oxygen content of 4 to 15 wt.-%, preferably 5 to 12 wt.-%

as measured by Energy-Dispersive X-ray spectroscopy (EDX) whereby the content of carbon, nitrogen and oxygen makes up 95 % to 100 % of the total mass of the second layer.

Typically the remainder to 100 % is at least mainly hydrogen.

Another aspect the invention relates to a carbon dioxide sensor material comprising

I) a substrate

II) carbon dioxide sensor composition according to the invention bound to the substrate via the second layer

as well as to a carbon dioxide sensing device comprising such carbon dioxide sensor material.

The invention further encompasses a process for the manufacture of the aforementioned carbon dioxide sensor compositions and sensor materials.

**Brief description of the drawings**

**Figure 1** shows photographs of various precursor materials employed to prepare carbon dioxide sensor materials according to the invention.

**Figure 2** shows a top view and side view illustration of the setup to perform step c) of the process to prepare carbon dioxide sensor materials inside a controlled atmosphere chamber.

**Figure 3** shows a photograph of a 20 cm$^2$ PET substrate with 36 LP-NC sensor strips.

**Figure 4** shows a top view SEM image of $LP_{O2}$-Ade380$_{90}$/Glu300$_{10}$ (example LP-14-e ($O_2$)).

**Figures 5 to 7** show top view SEM images with lower magnification of $LP_{N2}$-Ade380$_{90}$/Glu300$_{10}$ (example LP-14-g ($N_2$)), $LP_{air}$-Ade380$_{90}$/Glu300$_{10}$ (example LP-14-b (air)), and $LP_{O2}$-Ade380$_{90}$/Glu300$_{10}$ (example LP-14-e ($O_2$)) i.e. the same coated substrates exposed to a carbon dioxide laser under different gas atmospheres.

**Figure 8** shows a Raman spectrum of $LP_{N2}$-Ade380$_{90}$/Glu300$_{10}$ (example LP-14-g ($N_2$)) and $LP_{O2}$-Ade380$_{90}$/Glu300$_{10}$ (example LP-14-e ($O_2$) obtained upon excitation at 532 nm. The insets show a deconvolution of the D and G region.

**Figure 9** shows a cross sectional energy-dispersive X-ray analysis of $LP_{O2}$-Ade380$_{90}$/Glu300$_{10}$ a total thickness of 75 $\mu$m (example LP-14-e ($O_2$).

**Figures 10 to 12** show X-ray photoelectron spectrographs of the $O_{1s}$, $N_{1s}$, and $C_{1s}$ regions of of $LP_{N2}$-Ade380$_{90}$/Glu300$_{10}$ (example LP-14-g ($N_2$)), $LP_{air}$-Ade380$_{90}$/Glu300$_{10}$ (example LP-14-b (air)), and $LP_{O2}$-Ade380$_{90}$/Glu300$_{10}$ (example LP-14-e ($O_2$)).

**Figure 13** shows the resistive response of $LP_{O2}$-Ade380$_{90}$/Glu300$_{10}$ with varying thicknesses of 45, 75 and 100 $\mu$m (examples LP-14-d, e and f ($O_2$) using a mixed gas with 10% $CO_2$ and 90 % nitrogen as carrier gas).

**Figure 14** shows the resistive response of $LP_{O2}$-Ade380$_{90}$/Glu300$_{10}$ (example LP-14-f ($O_2$) towards exposure to different concentrations of carbon dioxide using nitrogen as carrier gas.

**Figure 15** shows the resistance response over multiple cycles of $LP_{O2}$-Ade380$_{90}$/Glu300$_{10}$ (example LP-14-f ($O_2$)) towards 10% of carbon dioxide using nitrogen as carrier gas or dry air as carrier gas.

**Figure 16** shows the resistance response of $LP_{O2}$-Ade380$_{90}$/Glu300$_{10}$ (example LP-14-f ($O_2$) towards using a mixed gas with 10% $CO_2$ and 90 % nitrogen as carrier gas at different relative humidities.

**Figure 17** shows the the curvature analysis of a strip of $LP_{O2}$-Ade380$_{90}$/Glu300$_{10}$ (example LP-14-f ($O_2$) in dependence of the distance between the two electrodes in the movable stage.

**Figure 18** shows the change in resistance upon steadily increasing the curvature of a strip of $LP_{O2}$-Ade380$_{90}$/Glu300$_{10}$.

**Detailed description of the Invention**

The invention also encompasses all combinations of all levels of preferred embodiments or parameter ranges as disclosed hereinafter either with each other or with the broadest disclosed parameter range or embodiment.

Whenever used herein the terms "including", "for example", "e.g.", "such as" and "like" are meant in the sense of "including but without being limited to" or "for example without limitation", respectively.

Whenever used herein the term "%" used for a gas, mixed gas or gas composition volume-% are meant.

As used herein the term "nitrogen-doped carbon material" generally denotes a material exhibiting at least one cumulative peak for nitrogen in a range of $400.0 \pm 3.0$ eV, one cumulative peak for carbon in a range of $286.0 \pm 4.0$ eV and one cumulative peak for oxygen in a range of $533.0 \pm 3.0$ eV each as measured by x-ray photoelectron spectroscopy (XPS) and with the elemental composition indicated above.

In one embodiment the at least one cumulative peak for nitrogen in the range of $400.0 \pm 3.0$ eV is observed as a result from overlapping peaks attributable to graphitic ($401.5 \pm 0.5$ eV) and/or pyridinic ($397.9 \pm 0.5$ eV) and/or pyrrolic nitrogen ($399.7 \pm 0.5$ eV).

In another embodiment the at least one cumulative peak for carbon in the range of $286.0 \pm 4.0$ eV is observed as a result from overlapping peaks attributable to $sp^2$ carbon-carbon bonds ($284.4 \pm 0.3$ eV) and/or $sp^3$ carbon-carbon bonds ($285.5 \pm 0.3$ eV), and/or C-N and/or C-O bonds ($286.2 \pm 0.3$ eV), and/or C=O and/or C=N bonds ($287.4 \pm 0.3$ eV) and/or a COOH group ($288.9 \pm 0.3$ eV).

In a further embodiment the at least one cumulative peak for oxygen in the range of $533.0 \pm 3.0$ eV is observed as a result from overlapping peaks attributable to O-C ($532.7 \pm 0.5$ eV) and/or O=C ($531.4 \pm 0.5$ eV) and/or O=C-O bonds ($533 \pm 0.5$ eV).

In one embodiment the carbon dioxide sensor composition shows at least three peaks at shifts of $1300 \pm 50$, $1600 \pm 50$ and $2400 \pm 50$ in the Raman Spectrum upon excitation at 532 nm. Such peaks are indicative for a graphitic or non-graphitic carbon with a high degree of defects.

The different layers of the carbon dioxide sensor composition are defined by their elemental composition. The elemental compositions are measured in accordance with the experimental part.

In one embodiment, the content of $sp^2$ carbon in the carbon dioxide sensor composition is from 40 to 60 wt.-% of the total carbon content, preferably from 45 to 60 wt.-% as measured by XPS.

In another embodiment, the content of pyrrolic oder imidazolic nitrogen in the carbon dioxide sensor composition is from 60 to 80 wt.-% of the total nitrogen content as measured by XPS.

In one embodiment the carbon dioxide sensor composition is present as a layered material having a total thickness of 20 to 150 $\mu$m, preferably 40 to 120 $\mu$m and more preferably 50 to 100 $\mu$m.

In one embodiment the first layer of the inventive carbon dioxide sensor composition has a thickness of 5 to 50 $\mu$m, preferably 5 to 40 $\mu$m and more preferably 10 to 40 $\mu$m.

In another embodiment the second layer of the inventive carbon dioxide sensor composition has a thickness of 15 to 145 $\mu$m, preferably 20 to 110 $\mu$m and more preferably 30 $\mu$m to 90 $\mu$m.

In one embodiment the first layer of the inventive carbon dioxide sensor composition makes up from 10 to 45 % and the second layer 55 to 90 % of the total thickness of the carbon dioxide sensor composition, preferably the first layer makes up 20 to 45 % and the second layer 55 to 80 % of the total thickness of the carbon dioxide sensor composition.

Typically, but depending on preparation conditions in some embodiments a gradual decrease in carbon content and a simoultaneous increase in nitrogen content is observed for the carbon sensor material from the first layer to the second layer.

In one embodiment the carbon dioxide sensor composition exhibits a conductivity of 0.8 to 6.0, preferably 1.0 to 4.0 S*cm$^{-1}$.

In one embodiment the carbon dioxide sensor composition exhibits a charge carrier density of $1.0*10^{18}$ to $5.0*10^{19}$ cm$^{-3}$, preferably of $5.0*10^{18}$ to $4.0*10^{19}$ cm$^{-3}$ as obtained by Hall measurement.

In one embodiment the carbon dioxide sensor composition exhibits a charge carrier density of 0.3 to 5.0 cm$^2$(V*s)$^{-1}$, preferably of 0.4 to 4.0 cm$^2$(V*s)$^{-1}$ as obtained by Hall measurement.

Elemental analysis and X-ray photoelectron spectroscopy (XPS), Energy-Dispersive X-ray spectroscopy (EDX), Hall measurements, Raman-Spectra and other methods applied to determine physical or chemical parameters of the carbon dioxide sensor compositions, the nitrogen-doped materials of the first layer or the second layer are described in more detail in the experimental section and all ranges and values of such parameters disclosed in this application are given herein with reference thereto.

It is known to those skilled in art, that due to their insolubility in commonly used media and their preparation process which typically involves a (laser-induced) thermolysis whereby organic precursors are condensed and/or decomposed and/or rearranged (hereinafter also referred to as "carbonization"), the inventive carbon dioxide sensor compositions in general and the first layer and the second layer of nitrogen-doped carbon materials in particular may have different properties depending on the preparation conditions and the starting materials chosen for their preparation.

The carbon dioxide sensor compositions may undergo oxidation or hydrolysis either during their preparation or thereafter when in contact with air and/or humidity. Therefore the inventive carbon nitride composition carbon dioxide sensor compositions typically also have a varying content of oxygen e.g.stemming from hydroxyl or carbonyl groups.

Where foaming agents such as carbohydrates or compounds derived therefrom are used the oxygen may also stem from these compounds.

For the reasons given above the carbon dioxide sensor compositions may alternatively or additionally also be specified by their preparation process.

In one embodiment the inventive carbon dioxide sensor compositions and sensor materials are prepared by a process comprising at least the steps of

    a) providing a starting material comprising

        i) at least one precursor material and

        ii) at least one film-forming polymer and

        iii) a diluent

    b) coating a substrate with the starting material and removing the diluent to obtain a coated substrate

    c) at least partially exposing the coating of the coated substrate obtained in step b) to electromagnetic irradiation having a wavelength of 780 nm to 1 mm, preferably 1,000 nm to 12,500 nm and an intensity and duration sufficient to induce at least partial carbonization of the coating

    d) optionally removing the coating prepared in step b) in cases where the coated substrate was only partially exposed to the electromagnetic irradiation.

In step a) a starting material comprising at least one precursor material, at least one film-forming polymer and a diluent is provided.

A suitable precursor material may be any material or compound that comprises at least one pyrrol or imidazole-ring.

Suitable precursor materials include those having a molecular mass of 500 g/mol or more.

In a preferred embodiment the number of pyrrol or imidazole-ring in the precursor material is such that the nitrogen content stemming from pyrrol or imidazole rings is from 5 wt.-% to 30 wt.-%, preferably from 8 to 30 wt.-%.

Its apparent to those skilled in the art that the upper limit of 30 wt.-% can only be obtained by polymeric imidazole materials while polymeric pyrrol materials exhibit a lower content of nitrogen as defined above.

Suitable precursor materials include homo- and copolymers comprising repeating units of one or more imidazole- or pyrrol- ring containing monomers such as 4-vinylimidazole, N-vinylimidazole and pyrrole. Such polymers include poly(4-vinylimidazole), poly(N-vinylimidazole), polypyrrole, for example undoped (without counterion) or doped (with counterions) polypyrroles. Doped polypyrrols include those having an oxidation degree between 0.25 and 0.5 with sulfonates e.g. methylsulfonate or benzosulfonate or p-tolylsulfonate as counterions.

Further suitable and even preferred precursor materials include precursor materials obtainable by the step of Pre-a) heating at least one precursor compound at a temperature of 300 to 400°C.

Heating to the indicated temperatures in step pre-a) can be accomplished in any device known to those skilled in the art and include heating in an oven, a muffle oven, a rotary kiln, a solar furnace or other devices of alike nature.

In on embodiment heating to the desired temperature is performed for a duration of 10 minutes to 24 h, preferably 30 minutes to 4 hours. Longer heating times are possible but add no value.

To achieve the desired temperature a temperature ramp from ambient temperature to the desired temperature can be applied. The heating rate may be from 0.5 K/minute to 100 K/minute, preferably from 2 K/minute to 10 K/minute.

Suitable precursor compounds have at least one pyrrol or imidazole-ring, a molecular mass of less than 500 g/mol and additionally at least one functional group selected from amino, imino and hydroxy.

Preferred precursor compounds include adenine, guanine, isoguanine, xanthine, hypoxanthine and 5-aminoimidazole, whereby adenine is particularly preferred.

The starting material further comprises at least one film-forming polymer.

As used herein a film-forming polymer is a polymer which can form a film on the substrate. The formation of a film can be demonstrated, for example, by looking at the substrate treated with the polymer under a microscope.

A film-forming polymer is typically a polymer with a molecular weight of at least 1,000 g/mol, preferably of at least 2,500 g/mol, particularly preferably of at least 5,000 g/mol, which comprises one or more repeating organic units.

The film-forming polymers of the present disclosure may be synthetically produced polymers which are manufactured by polymerization of one type of monomer or by polymerization of different types of monomers which are structurally different from each other. If the polymer is produced by polymerizing only one type of monomer, it is called a homopolymer. If structurally different monomer types are used in polymerization, the resulting polymer is called a copolymer.

The maximum molecular weight of the polymer depends on the degree of polymerization (number of polymerized monomers) and the batch size and is determined by the polymerization method. For the purposes of the present invention, it is preferred that the maximum molecular weight of the film-forming hydrophilic polymer is not more than $10^7$ g/mol, preferably not more than $10^6$ g/mol and particularly preferably not more than $10^5$ g/mol.

Preferred film-forming polymers are hydrophilic. As used herein hydrophilic means that the polymer exhibits a solubility of more than 1 % by weight, preferably more than 2 % by weight.

The water solubility of the hydrophilic film-forming polymer can for example be determined in the following way. 1.0 g of the polymer is placed in a beaker. Make up to 100 g with water. A stir-fish is added, and the mixture is heated to 25° C. on a magnetic stirrer while stirring. It is stirred for 60 minutes. The aqueous mixture is then visually assessed. A completely dissolved polymer appears macroscopically homogeneous. If the polymer-water mixture cannot be assessed visually due to a high turbidity of the mixture, the mixture is filtered. If no undissolved polymer remains on the filter paper, the solubility of the polymer is more than 1 % by weight.

Nonionic, anionic, and cationic polymers can be used as hydrophilic film-forming polymers.

Suitable hydrophilic film-forming polymers include polyvinylpyrrolidone (co)polymers, polyvinyl alcohol (co)polymers, vinyl acetate (co)polymers, carboxyvinyl (co)polymers, acrylic acid (co)polymers, methacrylic acid (co)polymers, natural gums, polysaccharides and/or acrylamide (co)polymers.

It is particularly preferred to use polyvinylpyrrolidone (PVP) and/or vinylpyrrolidone-containing copolymers as hydrophilic film-forming polymers.

Copolymers of polyvinylpyrrolidone include copolymers of vinylpyrrolidone with at least one further monomer selected from the group consisting of vinyl esters such as vinyl acetate, vinyl caprolactame, vinyl caprolactone, vinyl formamide, N-vinylimidazole, 4-vinylimidazole, vinyl acetate, styrene, ethylene, propylene, methacrylamides, acrylamides and vinyl alcohol.

Preferred hydrophilic film-forming polymers are polyvinylpyrrolidone, copolymers of N-vinylpyrrolidone and vinyl esters of carboxylic acids having 2 to 18 carbon atoms, of N-vinylpyrrolidone and vinyl acetate, copolymers of N-vinylpyrrolidone and N-vinylimidazole and methacrylamide, copolymers of N-vinylpyrrolidone and N-vinylimidazole and acrylamides and copolymers of N-vinylpyrrolidone with N,N-di($C_1$ to $C_4$)-alkylamino-($C_2$ to $C_4$)-alkylacrylamide.

Further suitable hydrophilic film-forming polymers include natural gums like xanthan gum, gellan gum and carob gum and polysaccharides like hydroxyethyl cellulose, hydroxypropyl cellulose, ethyl cellulose and carboxymethyl cellulose.

The starting material further comprises a diluent. A diluent can be any liquid compound or liquid mixture of compounds capable of at least partially, preferably fully dissolving the film-forming polymer. Thereby the diluent typically helps to reduce the viscosity of the starting material employed for the coating in step b).

It is apparent to those skilled in the art that depending on the type and amount of the components of the starting material, the precursor material(s), the film-forming polymer, and the optional components the most suitable diluents may vary. However the suitability of a diluent can be determined in very few orienting examples.

Typically, suitable diluents for example include organic diluents being liquid at 20°C. Such diluents include aliphatic and aromatic hydrocarbons which are optionally chlorinated such as toluene, xylene, n-hexane, cyclohexane, n-heptane, chlorobenzene and dichloromethane; ethers such as diethyl ether, tert.-butylmethyl ether, tert.-butylethyl ether, tetrahydrofurane and dioxane; esters like ethyl acetate, alcohols like monoalcohols and polyols. Such monoalcohols and polyols include methanol, ethanol, n-propanol, isopropanol, n-butanol, tert.-butanol, ethylene glycol, propylene glycol, 1,3-propanediol, diethylene glycol and 1,4-butanediol.

Further suitable diluents incluse nitriles such as benzonitrile and acetonitrile; amides such as formamide, N,N-dimethylformamide and N-methylpyrrolidone; sulfones such as dimethylsulfone; sulfoxides such as dimethylsulfoxide and to the extent miscible any mixture of all of the aforementioned diluents.

In one embodiment polyols, in particular ethylene glycol is employed as diluent.

The amount of precursor materials is for example selected such that the precursor materials make up 10 to 50 wt.-% of the sum of precursor materials, film-forming agents and diluent, preferably 20 to 50 wt.-%.

The amount of film-forming agent is for example selected such that the film-forming agent make up 5 to 30 wt.-% of the sum of precursor materials, film-forming agents and diluent, preferably 5 to 20 wt.-%.

The starting materials may optionally but preferably further comprise a foaming agent other than the precursor materials. As used herein a foaming agent denotes any material that is capable of liberating or forming gaseous compounds such as water, nitrogen or ammonia at the reaction conditions of step c) thereby aiding the formation of a porous structure of the resulting carbon dioxide sensor material.

Suitable foaming agents include mono-, oligo- or polysaccharides, preferably mono- or disacharides like glucose, fructose, galactose, xylose or foaming agents prepared by heating mono-, oligo- or polysaccharides at a temperature of 200 to 350°C, preferably to 250 to 320°C for at least 10 minutes, preferably for 30 minutes to 4 hours.

Where a foaming agent is employed in the starting material the amount of foaming agent is for example selected such that the ratio of the foaming agent to the precursor material is from 10:1 to 1:10, preferably 1:1 to 1:10 and more preferably 1:4 to 1:10.

The starting materials employed in step a) may further optionally comprise at least one inorganic salt, for example one or more alkali metal earth or alkali metal salts, preferably alkali metal salts such as nitrates, halides and sulfates, preferably halides, all of the aforementioned e.g. in form of nanoparticles. Preferred inorganic salts are alkali metal halides, whereby sodium iodide is particulary preferred.

Where an inorganic salt is employed in the starting material the amount of inorganic salt(s) is for example selected such that the weight ratio of the inorganic salt(s) to the precursor material(s) is from 1:20 to 1:1, preferably from 1:10 to 1:3.

The starting materials employed in step a) may further comprise or not an N-doped carbon precursor other than the precursor materials. Such N-doped carbon precursor other than the precursor materials may be any known material or compound that does not comprise imidazole oder pyrrol rings and can be converted into an N-doped carbon. Examples include urea, melamine, guanidine, cyanuric acid and dicyandiamide.

Where an N-doped carbon precursor other than the precursor materials is employed in the starting material the amount of N-doped carbon precursor is for example selected such that the weight ratio of the N-doped carbon precursor to the precursor material(s) is from 1:20 to 1:3, preferably from 1:20 to 1:5.

In a preferred embodiment the starting material forms a dispersion, preferably a homogeneous dispersion of the precursor material(s), the film-forming polymer(s), the diluents and the optional components employed.

Where desired or necessary homogenization can be aided by milling or grinding, for example by milling with a ball mill.

In step b), a substrate is coated with the starting material and the diluent is removed e.g. by evaporation. Evaporation may be effected by applying a vacuum and or heating the coated substrate to a suitable temperature for a sufficient time which can be easily determined by one skilled in the art.

A substrate might be any compound or material that is capable to withstand the conditions applied in step c) without substantial degradation or deformation.

Preferred substrates include polymers or any other material such as metal foils coated with a polymer whereby the polymers are solid up to 60°C, preferably up to 80°C and are virtually insoluble in water.

As used herein virtually insoluble in water means a water solubility of less than 0.10 g/l at 25°C. The solubility in water can for example be determined in analogy to the procedure described above for hydrophilic film-forming polymers.

Suitable polymers typically exhibit a molecular weight of at least 1,000 g/mol, preferably of at least 2500 g/mol, particularly preferably of at least 5,000 g/mol, and comprise one or more repeating organic units. The polymers of the present disclosure suitable as a substrate or coating may be synthetically produced polymers which are manufactured by polymerization of one type of monomer or by polymerization of different types of monomer which are structurally different from each other. If the polymer is produced by polymerizing only one type of monomer, it is called a homopolymer. If structurally different monomer types are used in polymerization, the resulting polymer is called a copolymer.

The maximum molecular weight of the polymer depends on the degree of polymerization (number of polymerized monomers) and the batch size and is determined by the polymerization method. For the purposes of the present invention, it is preferred that the maximum molecular weight of the polymer of or on the substrate is not more than $10^8$ g/mol.

Structurally the polymers may be any synthetic or natural or mixed natural and synthetic polymer, whereby polymers only comprising carbon and hydrogen and either additionally nitrogen and/or oxygen atoms or not are preferred.

Such polymers include polyethylene, polypropylene, polystyrene, polyesters like polycarbonates, polyethylene terephthalate and polybutylene terephthalate, polyacrylates and polymethylacrylates like polymethylmethacrylate, polyvinylacetals such as polyvinylbutyral, polyvinylesters such as polyvinylacetate and polyvinylpropionate, mixed polyvinylesters-polyvinylacetals; polyimides, natural rubber (NR), epoxidized natural rubber (ENR), polyisoprene rubber, polyisobutylene rubber, poly(styrene-co-butadiene) rubber (SBR), polybutadiene rubber (BR), ethylene vinylacetate (EVA) rubber, ethylene acrylate rubber, poly(isoprene-co-butadiene) rubber (IBR), styrene-isoprene-butadiene rubber (SIBR), ethylene-propylene rubber (EPR), ethylene-propylene-diene M-class rubber (EPDM), nitrile-butadiene rubber (NBR), hydrogenated nitrile-butadiene rubber (HNBR), propylene oxide polymers, butyl rubbers (isobutylene-co-isoprene rubber); poly(isobutylene-co-p-methylstyrene), poly(isobutylene-co-isoprene-co-styrene), polyurethanes (PU), polyacrylic esters (ACM, PMMA), thermoplastic polyester urethane (AU), thermoplastic polyether urethane (EU), polyamides such as PA 6 and PA 6,6, poly(3,4-ethylenedioxyselenophene) (PEDOS) and poly(3,4-ethylenedioxythiophene) (PEDOT).

In one embodiment the polymers are selected from polyethylene, polypropylene, polystyrene, polyimine, polyesters like polycarbonates, polyethylene terephthalate and polybutylene terephthalate, whereby polyethylene terephthalate is preferred.

The substrate typically has a thickness of 50 $\mu$m to 2.0 mm, preferably of 100 $\mu$m to 1.0 mm. Thicker substrates are possible but add no specific value and would reduce mechanical flexibility.

Step b) may be performed by any technique allowing to distribute a liquid or suspension on a substrate such as spraying, brushing, dripping, dipping, doctor-blading, spin-coating, whereby spin-coating or doctor-blading is preferred.

Evaporation of the diluent may be facilitated by applying reduced pressure or elevated temperatures each compared to ambient conditions.

Upon performance of step b) a coated substrate, is obtained whereby the coating essentially consists of the components of the starting material other than the diluent. The thickness of the coating is for example in the range of 20 to 150 $\mu$m, preferably in the range of 40 to 120 $\mu$m and more preferably in the range of 40 to 100 $\mu$m and typically virtually does not change after performing step c)

The thickness can be adjusted to a desired level for example by varying the ratio of diluent to the sum of film-forming polymers and the precursor materials. The less diluent is employed the more viscous the resulting coating usually is. Thickness can also be determined by the rate of spinning where spin-coating is applied or the number of spraying, brushing, dripping or dipping cycles.

The employment of film-forming polymer(s) typically leads to homogeneous, smooth coatings on the substrates.

In step c) the coating of the coated substrate obtained in step b) is at least partially exposed to electromagnetic irradiation with a wavelength of 780 nm to 1 mm with an intensity and for a duration sufficient to induce carbonization at the surface and/or within the coating.

Suitable light sources emitting electromagnetic radiation with a wavelength of 780 nm to 1 mm with an intensity sufficient to induce at least partial carbonization of the coating include infrared lasers such as a carbon dioxide laser having a wavelength of 9,400 and 10,600 nm or Neodymium doped yttrium-aluminum-garnet lasers (Nd-YAG) having a major wavelength of 1064 nm and further transitions at 946 nm, 1320 nm and 1444 nm.

Further suitable but less preferred infrared light sources are infrared LEDs; where the light is concentrated by lenses to achieve the desired intensity.

The intensity of the electromagnetic radiation sufficient to induce carbonization at the surface and/or within the coating is typically in the range of 500 W/cm$^2$ to 20,000 W/cm$^2$, preferably from 1,000 W/cm$^2$ to 5,000 W/cm$^2$.

The duration of irradiance may for example be from 5 ms to 0.5 s, preferably from 10 ms to 100 ms.

It is apparent to those skilled in the art that the thickness of the first layer and thus the electronic properties of the carbon dioxide sensor compositions and materials depend on the intensity and/or the duration of irradiance both together defining the total power input to the coating of the coated substrate.

The total power input typically is in the range of from 20 to 500 J/cm$^2$, preferably from 50 to 200 J/cm.

Step c) may be performed under substantial exclusion of oxygen or under an oxygen containing gas like air or pure oxygen. As used herein "substantial exclusion of oxygen" shall mean that the exposure of the coating of the coated substrate is performed under an inert gas or under a stream of inert gas or reduced pressure such that the partial pressure of oxygen in step c) is typically less than 10 hPa, preferably less than 5 hPa and more preferably less than 1 hPa. Suitable inert gases include nitrogen, argon and carbon dioxide, whereby nitrogen is preferred.

In a preferred emdiment step c) is performed under an oxygen containing gas comprising an oxygen partial pressure of 200 hPa or more, preferably 500 hPa or more and even more preferably 800 hPa or more.

The reaction pressure in step c) may for example be between 1 hPa and 5 MPa, preferably between 500 hPa and 1 MPa, for example 900 hPa to 1100 hPa.

Step c) can be carried out in any vessel suitable for that purpose. This includes open vessels having a gas connector to ensure the desired gas enviroment while performing step c). Typically the carbon dioxide sensor materials are then put into such vessels and irradiated with a suitable infrared light source.

Without wanting to be bound by theory it is assumed that the precursor material of the substrate coating undergoes a gradual conversion upon exposure to electromagnetic radiation carbonizes substantially to form the carbon rich first layer at the surface while the grade of carbonization gradually decreases with increasing distance from the surface forming the nitrogen rich second layer.

The first layer, due to its high content of carbon, is assumed to be responsible for the high conductivity and is also referred to as transducer layer. Due to its high nitrogen content the second layer is responsible for the good adsorption capabilities for carbon dioxide and is therefore also referred to as sensing layer.

It was found that the carbon dioxide sensor materials can be improved when the starting material comprises a foaming agent. This is likely attributable to the formation of a more porous structure of the carbon dioxide sensor material which not only facilitates accessability of carbon dioxide but also the number of accessible carbon dioxide binding sites within the second layer.

Further exposure to electromagnetic radiation under oxygen or an oxygen containing gas presumably supports the formation of oxygen-containing surface groups, such as C-O and C=O, and thereby increases the surface-polarity of the carbon dioxide sensor composition. Pyrrolic nitrogen species together with C=O as Lewis base groups might support a strong chemical interaction with $CO_2$, which explains why the exposure in step c) under oxygen or an oxygen containing gas promotes a higher response. Further carbonization and thus the formation of the carbon rich first layer (transducer layer) is likely supported. As a result the carbon dioxide sensor composition with its two layers or the sensor material respectively is formed in step c).

Oprional step d) i.e. removing the coating prepared in step b) in cases where the coated substrate was only partially exposed to electromagnetic radiation may be simply effected by washing or rinsing with a suitable solvent. A preferred solvent for this purpose is water.

The process described above describes a facile and efficient way to produce the inventive carbon dioxide sensor compositions and carbon dioxide sensor materials.

The invention therefore further encompasses carbon dioxide sensor materials comprising

A) a substrate

B) a carbon dioxide sensor composition according to the invention and bound to the substrate via the second layer.

The carbon dioxide sensor compositions and carbon dioxide sensor materials exhibit unusually high sensitivity for carbon dioxide for which the response defined as being the change in resistance after equilibration divided by the initial resistance ($\Delta R/R_0$) measured at a carbon dioxide content of 10 % in a carrier gas selected from the group consisting of nitrogen or air at a relative humidity is in the range of - 0.20 % to 20.00 %, preferably 0.20 % to 15.00 % and more preferably 1.00 % to 15.00 %.

The invention further relates to a carbon dioxide sensor device comprising at least

i) a first electrode

ii) a second electrode spaced from the first electrode

iii) a carbon dioxide sensor material according to the invention between the first electrode and the second electrode

iv) a system for applying a voltage to the carbon dioxide sensor material, and

v) electric circuitry including at least one measurement system in operative connection with the carbon dioxide sensor material to measure the electrical resistance or impedance relatable to the conductivity of the carbon dioxide sensor material which is dependent on the level of carbon dioxide at the carbon dioxide sensor material as analyte to be detected.

In one embodiment the carbon dioxide sensor device can be wearable or part of a wearable device.

As used herein the terms "electric circuitry including at least one measurement system in operative connection with the sensor" includes, but is not limited to, hardware, firmware, software or combinations of each to perform a function(s) or an action(s).

For example, electric circuitry may include a software controlled microprocessor, discrete logic such as an application specific integrated circuit (ASIC), or other programmed logic device.

The term "processor" as used herein includes, but is not limited to, one or more of virtually any number of processor systems or stand-alone processors, such as microprocessors, microcontrollers, central processing units (CPUs), and digital signal processors (DSPs), in any combination. The processor may be associated with various other circuits that support operation of the processor, such as random access memory (RAM), read-only memory (ROM), programmable read-only memory (PROM), erasable programmable read only memory (EPROM), clocks, decoders, memory controllers, or interrupt controllers, etc.

The term "control system" or "controller" as used herein includes, but is not limited to, any circuit or device that coordinates and controls the operation of, for example, one or more input or output devices. For example, a controller can include a device having one or more processors, microprocessors, or central processing units (CPUs) capable of being programmed to perform input or output functions.

The term "software" as used herein includes, but is not limited to, one or more computer readable or executable instructions that cause a computer or other electronic device to perform functions, actions, or behave in a desired manner. The instructions may be embodied in various forms such as routines, algorithms, modules or programs including separate applications or code from dynamically linked libraries. Software may also be implemented in various forms such as a stand-alone program, a function call, a servlet, an applet, instructions stored in a memory, part of an operating system or other type of executable instructions. It will be appreciated by one of ordinary skill in the art that the form of software is dependent on, for example, requirements of a desired application, the environment it runs on, or the desires of a designer/programmer or the like.

According to a further aspect of the invention there is provided a process for the detection or measurement of carbon dioxide levels in a gaseous environment by exposing a carbon dioxide sensor device according to the invention to such a gaseous environment

A major advantage of the present invention is the possibility to produce mechanically flexible, efficient (even in humid environments), highly responsive carbon dioxide sensor compositions and carbon dioxide sensors in a facile, very straight-forward process.

In the following, the present invention is illustrated by examples which however not intended to limit the scope of invention.

## Experimental section:

## I Chemicals and Methods

1 Scanning electron microscopy (SEM) energy-dispersive X-Ray (EDX) analysis

SEM was performed on a Zeiss LEO 1550-Gemini system (acceleration voltage: 3 to 10 kV). An Oxford Instruments X-MAX 80 mm$^2$ detector was used to collect the SEM-EDX data.

2 X-ray photoelectron spectroscopy (XPS)

X-ray photoelectron spectroscopy measurments were performed at the ISISS beamline of the synchrotron radiation facility BESSY II of Helmholtz-Zentrum Berlin, Germany. The used endstation consisted of a bending magnet (D41) and a plane grating monochromator (PGM) in the soft X-ray range 80 eV -2000 eV with a 80-200 $\mu$m beamspot. The photoelectron analyzer was provided by SPECS GmbH (Phoibos 150) hemispherical analyzer. In order to minimize losses of photons and electrons a 50 nm thick SiNx X-ray membrane close to the sample was used. Each sample was fixed on a sapphire sample holder.

XPS spectra were analyzed through CasaXPS and Igor Pro. The spectra were deconvoluted with a combined Gaussian and Lorentzian functions after a Shirley + linear background subtraction.

## 3 Raman Spectra

Raman spectra were obtained with a confocal Raman Microscope (alpha300, WITec, Germany) equipped with a piezo-scanner (P-500, Physik Instrumente, Karlsruhe, Germany). The laser, $\lambda$ = 532 nm was focused on the samples through a 50x objective. The laser power on the sample was set to 5.0 mW.

## 4 Hall measurements

The electrical conductivity $\sigma$ of carbonized films was determined at room temperature by the van der Pauw method in a Hall effect measurement system 8404 (Lake Shore Cryotronics, Inc.). The samples, all shaped in precise cloverleaf geometry with 10 mm diameter, were placed on a commercial 10 mm prober pin sample card of the 8404 Hall effect measurement system. Excitation currents from -5 to +5 mA have been used for the measurements..

Both, the DC and the Double AC Hall method employing two MFLI lock-in amplifiers (Zurich Instruments AG) and a CS580 voltage driven current Source (Stanford Research Systems) have been applied in the same setup to determine the charge carrier concentration. Different sets of oscillating excitation current (I =2 mA to 6 mA with frequency $f_I$= 88 Hz) and magnetic field (0.08 T, 0.16 T and 0.23 T with frequency $f_B$ = 0.8 Hz) have been used to measure the Hall voltage $V_H = V(f_I + f_B) + V(f_I - f_B)$, automated by a home made LabVIEW program. The linear relationship between $V_H$ and $I \times B$ (Eq. 1) is used to calculate the charge carrier density

$$p = \frac{1}{et}\frac{\partial(I \times B)}{\partial V_H}, \tag{1}$$

where e is the elementary charge and t is thickness of the sample.

The charge carrier mobility $\mu$ was calculated by the formula $\mu = \sigma/_{pe}$.

## 6 Chemicals and Substrates

D-glucose (anhydrous, Fisher Scientific GmbH), Adenine (>99%, TCI Europe N.V.), ethylene glycol (>99.7%, AnalaR Normapur, VWR Chemicals) and polyvinyl-pyrrolydone (average mol wt. 10,000, Sigma Aldrich) were used as received.

As a substrate polyethylene terephthalte sheets with a thickness of 170 $\mu$m (Melinex® sheets) were used.

### II Examples

### Examples 1 to 15 - Preparation of carbon dioxide sensor compositions and carbon dioxide sensor materials

### Step Pre-a): Preparation of precursor materials from precursor compounds

Precursor materials were prepared by loading 2 g of adenine as precursor compound into an alumina crucible or a quartz glass boat with a lid and heating at different temperatures between 300 and 400 °C in a tube furnace with a heating rate of 3 K· min$^{-1}$. A gentle stream of $N_2$ (0.1 L min$^{-1}$) was ensured during the reaction. The hold-time was 2h. The reaction products were retained and grinded in a ball mill (diameter of ball: 1cm) for 1h with a speed of 600 rpm to obtain finely powdered dark products.

The precursor material obtained by heating of Adenine a temperature of 300°C is hereinafter denoted as Ade300, a precursor material obtained by heating of Adenine at 320°C Ade320 etc.

1 g of D-Glucose was treated as described above at a temperature of 300°C to form a foaming agent which is hereinafter denoted as Glu300.

For comparison 2 g of cytosine was treated as described above at a temperature of 380°C to form a non inventive precursor material which is hereinafter denoted as Cyt380.

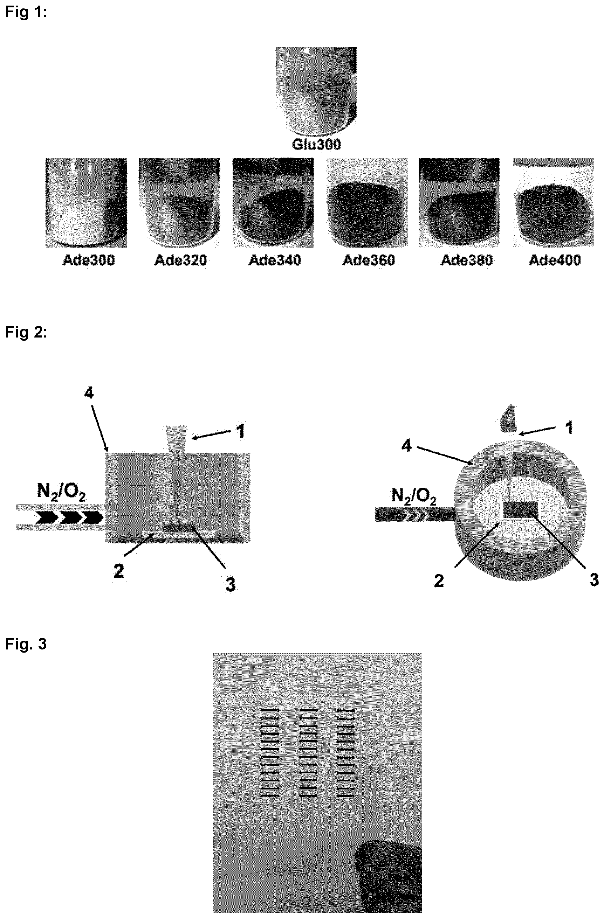

The elemental compositions of the precursor compound adenine and the precursor materials obtained according to step pre-a) and the foaming agent Glu300 and ist precursor D-Glucose are given in table 1. Photographs of the aforementioned materials and agents are shown in Figure 1

**Table 1**. Elemental composition of the precursor compounds and materials and the foaming agent as measured by combustion elemental analysis

| Elemental analysis of the precursor materials / foaming agent | | | | | |
|---|---|---|---|---|---|
| | C | N | O | H | C/N |
| Adenine (theoretical) | 44 | 52 | | 4 | 0.84 |
| Ade300 | 43 | 45 | 3 | 4 | 0.95 |
| Ade320 | 45 | 43 | 7 | 3 | 1.04 |
| Ade340 | 46 | 44 | 6 | 3 | 1.06 |
| Ade360 | 45 | 42 | 7 | 3 | 1.07 |
| Ade380 | 46 | 42 | 7 | 3 | 1.09 |
| Ade400 | 47 | 43 | 7 | 3 | 1.09 |
| D-Glucose (theoretical) | 40 | | 53 | 7 | |
| Glu300 | 63 | | 31 | 5 | |

### Step a): providing the starting material

Polyvinylpyrrolidone (PVP) as a film-forming agent was dissolved in a ethyleneglycol as a diluent to obtain a 0.2 g mL$^{-1}$ solution (PVP/ethyleneglycol). Sodium iodide (0.4g/mL) was added and dissolved. The solution was added to the precursor material and optionally the foaming agent Glu300 obtained in step pre-a) in the amounts shown in table 1 and gently stirred for 24 h to obtain the starting materials as homogeneous dispersions. All concentrations of the different samples with respect to the volume of the diluent are given in table 2.

**Table 2:** The composition of the starting materials

| Example | Starting Material Composition (Precursor Compound/ Foaming Agent) | Precursor compound [g·mL$^{-1}$]* | Glu300 [g·mL$^{-1}$]* | Ethylene glycol / PVP $\mu$L |
|---|---|---|---|---|
| 1 | Ade300 / none | 0.57 | 0 | 350 |

(continued)

| Example | Starting Material Composition (Precursor Compound/ Foaming Agent) | Precursor compound $[g \cdot mL^{-1}]^*$ | Glu300 $[g \cdot mL^{-1}]^*$ | Ethylene glycol / PVP $\mu L$ |
|---|---|---|---|---|
| 2 | Ade320 / none | 0.57 | 0 | 350 |
| 3 | Ade340 / none | 0.57 | 0 | 350 |
| 4 | Ade360 / none | 0.57 | 0 | 350 |
| 5 | Ade380 / none | 0.57 | 0 | 350 |
| 6 | Ade400 / none | 0.57 | 0 | 350 |
| 7 (for comparison) | None / Glu300 | 0 | 0.47 | 350 |
| 8** | $Ade380_{10}$/ $Glu300_{90}$ | 0.06 | 0.51 | 350 |
| 9** | $Ade380_{20}$/ $Glu300_{80}$ | 0.11 | 0.46 | 350 |
| 10** | $Ade380_{35}$/ $Glu300_{65}$ | 0.2 | 0.37 | 350 |
| 11** | $Ade380_{50}$/ $Glu300_{50}$ | 0.29 | 0.29 | 350 |
| 12** | $Ade380_{65}$/ $Glu300_{35}$ | 0.37 | 0.2 | 350 |
| 13** | $Ade380_{80}$/ $Glu300_{20}$ | 0.45 | 0.12 | 350 |
| 14** | $Ade380_{90}$/ $Glu300_{10}$ | 0.51 | 0.06 | 350 |
| 15** (for comparison) | $Cyt380_{90}$ / $Glu300_{10}$ | 0.51 | 0.06 | 350 |

\* $[g \cdot mL^{-1}]$ denotes grams of precursor material or foaming agent per ml of PVP/ethylene glycol mixture.
\*\* In examples 8 to 14 the lower script number at the precursor material and the foaming agent indicates the weight content of the respective components with respect to the sum of precursor material and the foaming agent.

**Step b):** Coating of the substrate

Around 1 ml of the starting material was applied onto the substrate (20 cm$^2$ of a polyethylene terephthalate sheet with a thickness of 170 $\mu$m and doctor bladed with a blade-distance between 300 and 500 $\mu$m. Ethylene glycol was then evaporated at 80°C on a precision hotplate (PZ2860-SR, Gestigkeit GmbH) to obtain the coated substrates with coating thicknesses between 45 to 100 $\mu$m. The resulting thickness was determined with a digital micrometer or vertical scanning interferometry.

For examples starting materials 1 to 13 i.e. experiments C-1 to C-13 (with C indicating the step b) of coating) the coating thickness was 75 $\mu$m, for example 14 three different blade distances were applied to obtain the coated substrates C-14-a with a coating thickness of 45 $\mu$m , C-14-b with a coating thickness of 75 $\mu$m and C-14-c with a coating thickness of 100 $\mu$m. Comparison example C-15 was also prepared with a coating thickness of 100 $\mu$m. After step c) the thickness of the carbon dioxide sensor material was unchanged compared to the coating employed.

**Step c): Exposure of the coated substrate to IR-electromagnetic radiation to induce at least partial carbonization of the coating**

The coated substrates obtained in step b) were irradiated with a $CO_2$-laser under different atmospheres (air, $N_2$, or $O_2$) to create the carbon dioxide sensor material. In each batch, 36 strips of carbon dioxide sensor materials were produced.

As the $CO_2$-laser a high-precision laser engraver setup (Speedy 100, Trotec) equipped with a 60 W carbon dioxide laser was used. Focusing was achieved with a 2.5 inch focus lens providing a focal depth of ~ 3 mm and a focus diameter of 170 $\mu$m. The center wavelengths of the laser was 10.6 $\pm$ 0.03 $\mu$m. The scanning speed v', generically given in %, was converted into $s \cdot m^{-1}$. The effective output power P of the laser was measured with a Solo 2 (Gentec Electro-Optics) power meter. The resulting energy input per distance (or fluence) F in $J \cdot m^{-1}$ in the vector mode onto the coating of the coated substrate is given by

$$F = P \cdot v'$$

For the experiments, the laser settings were adjusted to meet the requirements of the carbon dioxide sensor compositions according to table 3.

A standard carbon dioxide sensor material was prepared to comprise five parallel lines of 5 mm in length distributed across a width of 0.5 mm.

An open-top atmospheric chamber was designed as depicted in **Figure 2** to generate a continuous flow of a selected gas ($O_2$ and/or $N_2$) for the fabrication of the carbon dioxide sensor material under a controlled gas atmosphere. In **Figure 2** the reference numbers denote the laser source 1, the substrate 2, the coating 3 and a ring shaped chamber 4 with an entry for nitrogen and/or oxygen.

To optimize the laser parameters selected experiments were made with selected combinations of precursor materials / foaming agents under air (79 % $N_2$ / 21 % $O_2$). The results are shown in table 3.

**Table 3**. Laser parameters used in selected experiments

| Example / Coated Substrate (Precursor Material / Foaming Agent) | Coating Thickness $\mu$m | Power % (gen.) | Power W | Speed % (gen.) | Speed s·m$^{-1}$ | Fluence J·m$^{-1}$ |
|---|---|---|---|---|---|---|
| LP-7(from C-7 / Glu300) | 75 | 2.20 | 1.12 | 0.30 | 189 | 212 |
| LP-5 (from C-5 / Ade380) | 75 | 2.10 | 1.07 | 0.30 | 189 | 202 |
| LP-14-a (from C14-a) (Ade380$_{90}$/Glu300$_{10}$) | 45 | 1.90 | 0.97 | 0.30 | 189 | 183 |
| LP-14-b (from C-14-b) (Ade380$_{90}$/Glu300$_{10}$) | 75 | 2.10 | 1.07 | 0.30 | 189 | 202 |
| LP-14c (from C-14-c) (Ade380$_{90}$/Glu300$_{10}$) | 100 | 2.20 | 1.12 | 0.30 | 189 | 212 |

| Example (Precursor material / Foaming Agent) | Irradiation time [ms] | | Irradiation intensity [W/cm$^2$] | | Energy applied [J/cm2] | |
|---|---|---|---|---|---|---|
| LP-7 (from C-7) ( - / Glu300) | 32 | | 3875 | | 124.7 | |
| LP-5 (from C-5) (Ade380 / - ) | 32 | | 3700 | | 118.8 | |
| LP-14-a (from C-14-a) (Ade380$_{90}$/Glu300$_{10}$) | 32 | | 3350 | | 107.6 | |
| LP-14-b (from C-14-b) (Ade380$_{90}$/Glu300$_{10}$) | 32 | | 3700 | | 118.8 | |
| LP-14-c (from C-14-c) (Ade380$_{90}$/Glu300$_{10}$) | 32 | | 3875 | | 124.7 | |

In table 3 "LP" means Laser patterned, the number following LP indicates the number of the experiment of which the starting material and the coated substrate was taken (see table 2).

With the same Laser parameters of Example LP-14-c performed under air further coated substrates with a thickness of 75 $\mu$m and 100 $\mu$m were laser patterned under oxygen and nitrogen respectively.

These are denominated as:

Example LP-14-d ($O_2$) or LP$_{O2}$-Ade380$_{90}$/Glu300$_{10}$ with a thickness of 45 $\mu$m,
Example LP-14-e ($O_2$) or LP$_{O2}$-Ade380$_{90}$/Glu300$_{10}$ with a thickness of 75 $\mu$m,
Example LP-14-f ($O_2$) or LP$_{N2}$-Ade380$_{90}$/Glu300$_{10}$ with a thickness of 100 $\mu$m and
Example LP-14-g ($N_2$) or LP$_{O2}$-Ade380$_{90}$/Glu300$_{10}$ with a thickness of 75 $\mu$m,
Example LP-14-h ($N_2$) or LP$_{N2}$-Ade380$_{90}$/Glu300$_{10}$ with a thickness of 100 $\mu$m.

For comparison, the coated substrate of example C-15 was treated identically (example LP-15 ($O_2$) or LP$_{O2}$-Cyt380$_{90}$/Glu300$_{10}$.

**Step d): Removal of the coating**

In order to remove the part of the coating that was not exposed to irradiation, the carbon dioxide sensor material was rinsed with deionized water several times and dried. The resulting carbon dioxide sensor materials are shown as photograph in **Figure 3**.

**Characterization**

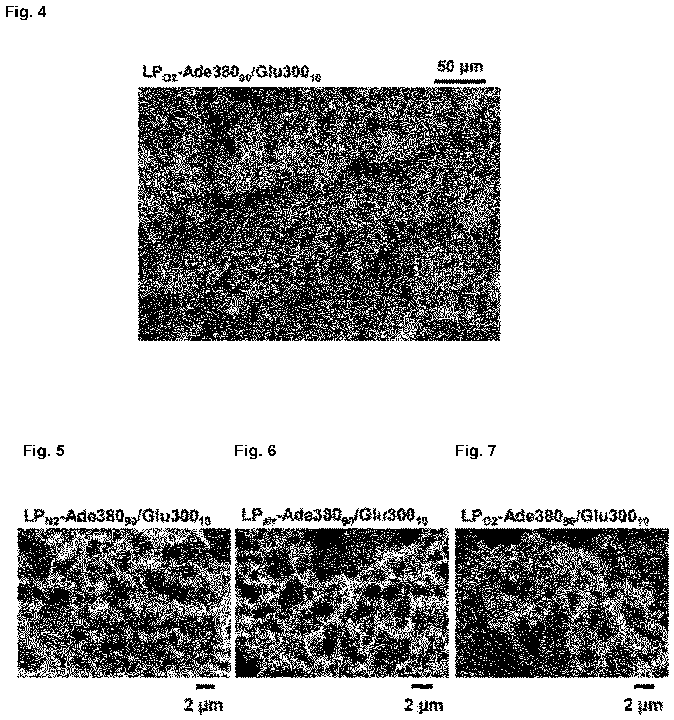

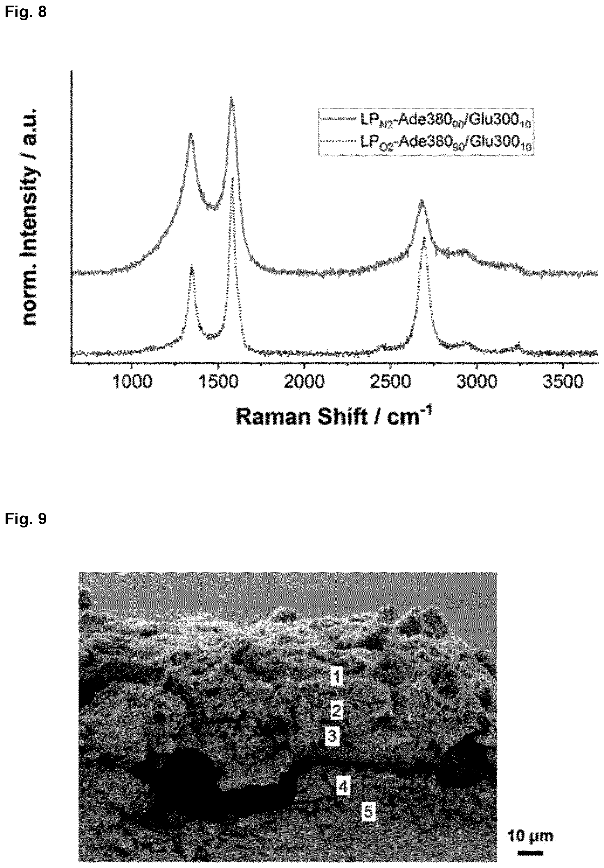

One of the best carbon dioxide sensor materials (example LP-14-d ($O_2$) - $LP_{O2}$-Ade380$_{90}$/Glu300$_{10}$) was morphologically and chemically investigated in detail. Throughout the entire laser-irradiated area the carbon dioxide sensor composition shows the typical porous morphology obtained upon laser-treatment (see **Figure 4)**. This morphology was obtained independent of the reaction atmosphere. However, under oxygen a crumpled surface structure with feature sizes on the order of 100 to 300 nm on top of the composition appears, which are likely attributable to the enhanced combustion process in the presence of oxygen (see **Figures 5 to 7)**. This, in turn, leads to an enhanced carbonization process on the surface. For all reaction atmospheres, the Raman spectra taken on the top of the compositions show common features of a turbostratic graphitic material, i.e. the presence of sharp D-, G-, and D'-bands, indicating a high degree of carbonization on top of the films (see **Figure 8)**.

Notably, the carbon dioxide sensor composition obtained in oxygen atmosphere, $LP_{O2}$-Ade380$_{90}$/Glu300$_{10}$ , shows a significantly higher degree of carbonization as only negligible contributions of sp$^3$- and disorder-related D4 and D3 bands at 1200 and 1460 cm$^{-1}$, respectively, and a lower defect-attributed D-band compared to $LP_{N2}$-Ade380$_{90}$/Glu300$_{10}$ (example LP-14-e ($N_2$) are observed (see interpretation of Raman spectra in [25]D. B. Schüpfer, F. Badaczewski, J. Peilstöcker, J. M. Guerra-Castro, H. Shim, S. Firoozabadi, A. Beyer, K. Volz, V. Presser, C. Heiliger, B. Smarsly, P. J. Klar, "Monitoring the thermally induced transition from sp3-hybridized into sp2-hybridized carbons" Carbon N. Y. 2021, 172, 214-227 and [26] M. Pawlyta, J.-N. Rouzaud, S. Duber, "Raman microspectroscopy characterization of carbon blacks: Spectral analysis and structural information" Carbon N. Y. 2015, 84, 479-490).

The formation of a structural and chemical gradient across the layer structure of the carbon sensor composition was observed. The exposure to the infrared laser light resulted in a lower degree of carbonization below a depth of 20 to 30 $\mu$m. This carbonization gradient is visualized in the cross-sectional EDX elemental maps of the $LP_{O2}$-Ade380$_{90}$/Glu300$_{10}$ composition presented in Figure 8. The chemical composition changes from 85 wt% C, 6 wt% N, and 9 wt% O on top of the first layer to 55 wt%, 40 wt% N, and 5 wt% O at the bottom of the second layer. The elemental composition as measured by SEM-EDX for positions 1 to 5 as shown in **Figure 9** are shown in table 4.

The thickness of thr first layer was 30 $\pm$ 2 $\mu$m, the thickness of the second layer 45 $\pm$ 2 $\mu$m.

Table 4: Elemental compositions at the positions 1 to 5 of $LP_{O2}$-Ade380$_{90}$/Glu300$_{10}$ as shown in **Figure 9.**

| Position | C [wt.-%] | N [wt.-%] | O [wt.-%] |
|---|---|---|---|
| 01 | 85 | 6 | 9 |
| 02 | 84 | 10 | 6 |
| 03 | 65 | 30 | 5 |
| 04 | 55 | 35 | 10 |
| 05 | 55 | 40 | 5 |

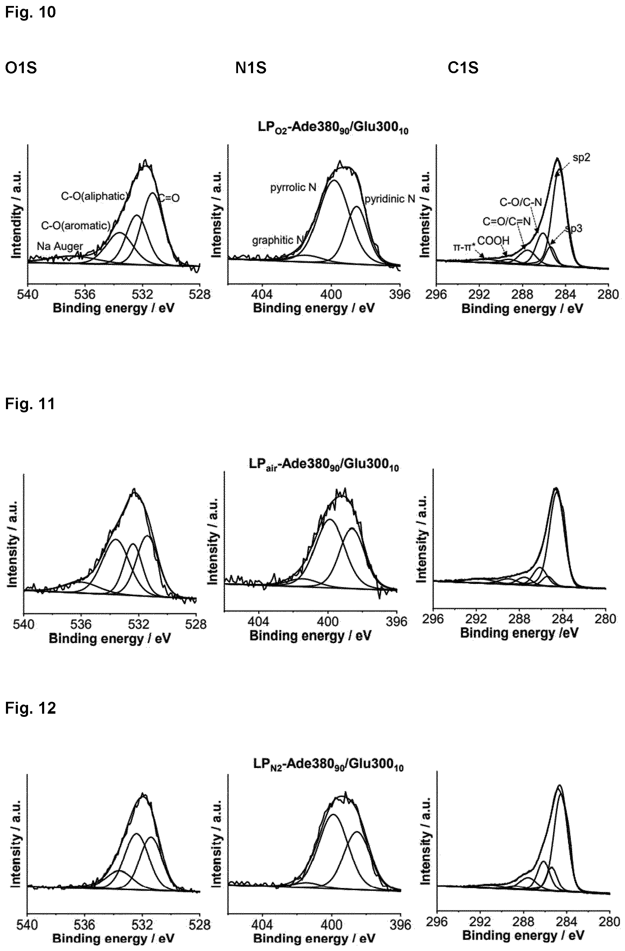

An important asset of the inventive carbon dioxide sensor compositions described herein are their nitrogen functionalities. To understand the chemical composition of the most active samples $LP_{O2}$-Ade380$_{90}$/Glu300$_{10}$, X-ray photoelectron spectroscopy was performed. The carbon dioxide sensor compositions were characterized by a high degree of carbonization with a number of functional groups confirmed by the prominent sp$^2$-carbon peak at 284.7 in the $C_{1s}$ region and signals at 285.9, 286.7 and 288.2eV assigned to sp$^3$-carbon, C-N/C-O, and C=N/C=O, respectively. Oxygen is bound in the form of C=O, C-O and O=C-O as evidenced by a set of three peaks at 531.2, 532.3 and 533.8 eV, respectively. Most importantly, the $N_{1s}$ area shows a prominent signal at 399.8 eV stemming from pyrrolic or imidazolic N and two minor peaks at 398.5 and 401.5 eV typical for pyridinic and graphitic N, respectively (see **Figures 10 to 12**). As shown in table 5, among the nitrogen functionalities, the pyrrolic/imidazolic nitrogen amounts for 61 wt.-% in $LP_{O2}$-Ade380$_{90}$/Glu300$_{10}$. This composition is independent of the addition of Glu300 as a foaming agent as it is also observed for the analogous material prepared without Glu300 (example LP-5 ($O_2$), $LP_{O2}$-Ade380).

For the carbon dioxide sensor materials according to examples LP-14-e ($O_2$) and LP-14-g ($N_2$) the conductivities , charge carrier densities and charge carrier mobilities were obtained by Hall measurement. The reuslts are given in table 5.

**Table 5:** Charge carrier properties

| Sample | conductivity ($\sigma$) | density (p) | mobility ($\mu$) |
|---|---|---|---|
| | $S \cdot m^{-1}$ | $\times 10^{-25} m^{-3}$ | $\times 10^{-5} m^2 \cdot (V \cdot s)^{-1}$ |
| $LP_{N2}$-Ade380$_{90}$/Glu300$_{10}$ | 58 ± 0.6 | 0.82 ± 0.063 | 4.4 ± 0.38 |
| $LP_{O2}$-Ade380$_{90}$/Glu300$_{10}$ | 89 ± 0.9 | 1.2 ± 0.051 | 4.6 ± 0.24 |

**Example 16: Carbon dioxide sensing**

For the suitability as chemiresistive sensors, the electric conductivity ($\sigma$) and its change upon exposure of $CO_2$ of the carbon dioxide sensing materials (response: $\Delta R/R_0$) are essential factors. For the carbon dioxide sensing materials LP-14-c (air) / , LP-14-d,e,f ($O_2$) and LP-14-g,h ($N_2$) the response to exposure of $CO_2$ of these was evaluated.

To do this the carbon dioxide sensor materials obtained as described above were placed in a gas-proof glass flow-cell ($V_{cell} \approx$ 0.1 L) and their ends were connected to two electrode bins. The electrical characterization was performed with an impedance unit at a frequency of 80 Hz and an alternating current (AC) of 0.05 mA for all measurements. The total flow of gas mixtures from a gas inlet into the sensor chamber was set to 1 L-min$^{-1}$ whereby the carbon dioxide levels were adjusted to 0.5 %, 1%, 5% and 10% $CO_2$ (with respect to the total flow) by mass flow controllers (Brooks Instrument GmbH) using nitrogen gas or dry air as carrier gas.

Langmuir's model was used to describe the interaction between the gas molecules and the sensor surface. At a given temperature, the fraction of occupied sites on the sensor surface is $\theta$. The equilibrium constant of adsorption K is defined as the ratio between the rate constants of adsorption $k_A$ and desorption $k_D$. The variation of $\theta$ is given by eq (1), where $P_A$ is the partial pressure of the analyte in the gas phase:

$$\theta = \frac{K * P_A}{1 + K * P_A} \qquad (1)$$

When molecules are adsorbed by the carbon dioxide sensor material according tot he invention, the resistivity decreases. Then $\theta$ is given by:

$$\theta = \alpha * (R_0 - R_{eq}) \qquad (2)$$

where $R_0$ is the initial resistance, and $R_{eq}$ is the resistance at equilibrium, $\alpha$ is the proportionality coefficient.

The results are shown in table 6.

Table 6: Response of various carbon dioxide sensor materials obtained by using the laser parameters of example LP-14-c and different atmospheres during step c) each having a thickness of 75 $\mu$m.

| Example / Starting material | | $\Delta R/R$ / % / Atmossphere employed in step c) | | |
|---|---|---|---|---|
| | | $N_2$ (-a/-g) | Air (-b/b2) | $O_2$ (c/e) |
| LP-5-a, -b, -c | Ade380 / none | -0.22 (-a) | -0.4 | -3.2 |
| LP-8-a, -b, -c | Ade380$_{10}$/ Glu300$_{90}$ | -0.21 | -0.5 | -6.3 |
| LP-9-a, -b, -c | Ade380$_{20}$/ Glu300$_{80}$ | -0.19 | -0.4 | -5.0 |
| LP-10-a, -b, -c | Ade380$_{35}$/ Glu300$_{65}$ | -0.56 | -0.7 | -0.39 |
| LP-11-a, -b, -c | Ade380$_{50}$/ Glu300$_{50}$ | -0.15 | -0.31 | -0.39 |
| LP-12-a, -b, -c | Ade380$_{65}$/ Glu300$_{35}$ | -0.19 | -0.18 | -0.23 |
| LP-13-a, -b, -c | Ade380$_{80}$/ Glu300$_{20}$ | -0.22 | -0.3 | -0.17 |
| LP-14-g; -b2; -e | Ade380$_{90}$/ Glu300$_{10}$ | -0.26 | -2.0 | -0.23 |

(continued)

| Example / Starting material | | $\Delta R/R$ / % / Atmossphere employed in step c) | | |
| --- | --- | --- | --- | --- |
| | | $N_2$ (-a/-g) | Air (-b/b2) | $O_2$ (c/e) |
| LP-7-a, -b, -c (for comparison) | none / Glu 300 | -0.15 | -0.15 | -0.15 |

As can been taken from table 6 the best response at a thickness of 75 $\mu$m as regards composition is obtained for $LPO_2$-Ade380$_{90}$/Glu300$_{10}$) reaching $\Delta R/R_0$ = -6.3 %.

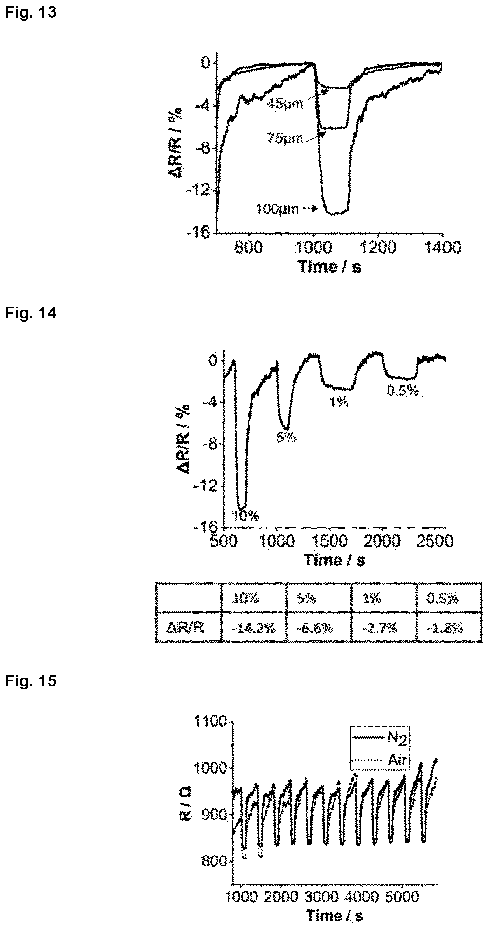

For the analogous material with a thickness of 100 $\mu$m a response of even $\Delta R/R_0$ = -14.3%. was obtained while the thinner material with a thickness of 45 $\mu$m showed less response (see **Figure 13).**

For comparison the pure glucose derived materials were tested, but all samples regardless of the atmosphere employed (LP-7 ($N_2$/air/$O_2$) or LP$_{N2/air/O2}$-Glu300) show a low response of only $\Delta R/R_0$ = - 0.15 %.

For further comparison, no response to exposure of 10% $CO_2$ whatsoever was detected for LP-15 ($O_2$) made from cytosine as a precursor. Laser-treatment of cytosine produces highly-nitrogen doped compositions, but the absence of imidazole or pyrrole- rings in the second layer presumably brings forth different N-sites in the respective material which are insensitive to carbon dioxide.

**Varying the carbon dioxide levels**

The carbon dioxide sensor material according to example LP-14-d ($O_2$) (LP$_{O2}$-Ade380$_{90}$/Glu300$_{10}$ ) was further tested with regard to ist response to different concentrations of $CO_2$. As can be seen in **Figure 14** the response follows a follows a linear trend, indicating that no saturation is reached at the maximum tested analyte concentration of 10 %. At low concentrations of 0.5% $CO_2$ still a good response of $\Delta R/R_0$ = -1.8% is measured. The response is stable for a series of cycles and is identical in air or $N_2$ as carrier gas (see **Figure 15).** The response and recovery time during $CO_2$ cycling is the same in both environments with $t_{res}$ = 25 s and $t_{rec}$ = 60s, respectively. This indicates that the presence of $O_2$ does not influence the detection mechanism.

**Testing in humid environments**

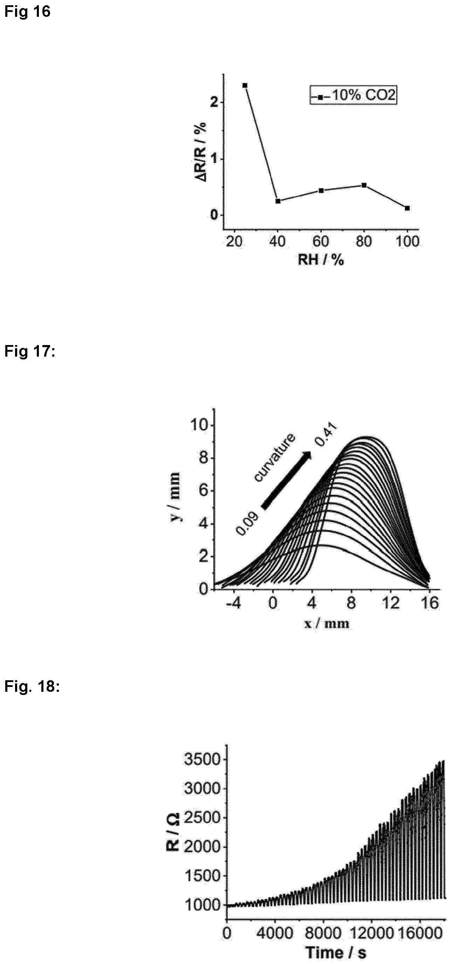

The sensing performance of the carbon sensor material according to example LP-14-d ($O_2$) (LP$_{O2}$-Ade380$_{90}$/Glu300$_{10}$) was further tested at different relative humidities in the same setup. Naturally, the polar surface of the material shows a high affinity to adsorption of $H_2O$. It is noteworthy that an obvious and stable response to $CO_2$ at high relative humidities is still detectable. For example, in the range of RH = 40-80%, the response increases from $\Delta R/R_0$ = 0.25 to 0.53% (see **Figure 16).** This shows that even in a high relative humidity, the carbon dioxide sensor material according to the invention shows a selective response to adsorption of $CO_2$.

**Example 17: Bending experiments**

The experiments were performed with a home-made movable stage. Carbon dioxide sensor material according to the invention was mounted between two electrodes (one movable) in a distance of 25 mm and bent in positive (upwards) direction (see **Figure 17)**. To provide a better electrical contact both ends of the carbon strip were connected with silver paint. The curvature of the bending was determined by translating the shapes from photographs into x-y data using the freeware Engauge Digitizer. The curvature is defined as the inverse radius at the maximum point of bending.

The curvature of the bending is dependent on the distance between the two electrodes and was determined in a range between a minimum value of 0.09 and a maximum value of 0.41 mm$^{-1}$. The response changes gradually with the curvature. During the first bending sequence, the sensor strip was bent at a slow speed of only 10 $\mu$m s$^{-1}$ starting at low curvatures of 0.09 mm$^{-1}$ and slowly increasing to the maximum curvature of 0.41 mm$^{-1}$ (three times for each curvature/amplitude). **Figure 18** shows the corresponding response of increasing R: small change at low curvatures, becomes larger at increasing curvatures. A quantitative response to any curvature was observed showing the huge mechanical flexibility of the carbon dioxide sensor materials according to this invention.

**Claims**

1. A carbon dioxide sensor composition comprising at least

1) a first layer comprising a nitrogen-doped carbon material having

    a) a carbon content of 75 to 95 wt-%, preferably of 75 to 90 wt.-% and
    b) a nitrogen content of 2 to 15 wt.-%, preferably 5 to 15 wt.-% and
    c) an oxygen content of 3 to 12 wt.-%, preferably 5 to 10 wt.-%

as measured by Energy-Dispersive X-ray spectroscopy (EDX) whereby the content of carbon, nitrogen and oxygen makes up 98 % to 100 % of the total mass of the first layer and
2) a second layer comprising a nitrogen-doped carbon material having a

    a) carbon content of 45 to 74 wt-%, preferably of 50 to 74 wt.-% and
    b) a nitrogen content of 22 to 45 wt.-%, preferably 23 to 42 wt.-% and
    c) an oxygen content of 4 to 15 wt.-%, preferably 5 to 12 wt.-%

as measured by Energy-Dispersive X-ray spectroscopy (EDX) whereby the content of carbon, nitrogen and oxygen makes up 95 % to 100 % of the total mass of the second layer.

2. The carbon dioxide sensor composition according to claim 1 having a total thickness of 20 to 150 $\mu$m, preferably 40 to 120 $\mu$m and more preferably 50 to 100 $\mu$m.

3. The carbon dioxide sensor composition according to claim 1 or 2, wherein the first layer makes up from 10 to 45 % and the second layer 55 to 90 % of the total thickness of the carbon dioxide sensor composition.

4. The carbon dioxide sensor composition according to any one of claims 1 to 3 having a conductivity of 0.8 to 6.0, preferably 1.0 to 4.0 S*cm$^{-1}$.

5. A carbon dioxide sensor material comprising

    I) a substrate
    II) a carbon dioxide sensor composition according to anyone of claims 1 to 4 and bound to the substrate via the second layer.

6. A process for the prepration of carbon dioxide sensor compositions, preferably those according to claims 1 to 4, or carbon dioxide sensor materials, preferably those according to claim 5, the process comprising at least the steps of

    a) providing a starting material comprising

        i) at least one precursor material being a material or compound that comprises at least one pyrrol or imidazole-ring and having a molecular mass of 500 g/mol or more and
        ii) at least one film-forming polymer and
        iii) a diluent

    b) coating a substrate with the starting material and removing the diluent to obtain a coated substrate
    c) at least partially exposing the coating of the coated substrate obtained in step b) to electromagnetic irradiation having a wavelength of 780 nm to 1 mm, preferably 1,000 nm to 12,500 nm and an intensity and duration sufficient to induce at least partial carbonization of the coating
    d) optionally removing the coating prepared in step b) in cases where the coated substrate was only partially exposed to the electromagnetic irradiation.

7. A process according to claim 6, wherein the precursor material is obtainable by the step of

    Pre-a) heating at least one precursor compound at a temperature of 300 to 400°C whereby the precursor compound(s) comprise at least one pyrrol or imidazole-ring,
    a molecular mass of less than 500 g/mol and additionally at least one functional group selected from amino, imino and hydroxy.

8. A process according to claim 7, wherein the precursor compounds are selected from adenine, guanine, isoguanine, xanthine, hypoxanthine and 5-aminoimidazole, whereby adenine is particularly preferred.

9.  A process according to anyone of claims 6 to 8, wherein the film-forming polymer(s) are hydrophilic.

10. A process according to anyone of claims 6 to 9, wherein the starting materials further comprise a foaming agent other than the precursor material(s).

11. A process according to anyone of claims 6 to 10, wherein the starting materials further comprise at least one inorganic salt.

12. A process according to anyone of claims 6 to 11 wherein the substrate is a polymer or any other material coated with a polymer whereby the polymers are solid up to 60°C, preferably up to 80°C and have a water solubility of less than 0.10 g/l at 25°C. In step c) the coating of the coated substrate obtained in step b) is at least partially exposed to electromagnetic irradiation with a wavelength of 780 nm to 1 mm with an intensity and for a duration sufficient to induce carbonization at the surface and/or within the coating.

13. A process according to anyone of claims 6 to 12, wherein in step c) a carbon dioxide laser having a wavelength of 9,400 and 10,600 nm or Neodymium doped yttrium-aluminum-garnet lasers (Nd-YAG) having a major wavelength of 1064 nm and further transitions at 946 nm, 1320 nm and 1444 nm are employed.

14. A process according to anyone of claims 6 to 13, wherein step c) is performed in the presence of an oxygen containing gas like air or oxygen.

15. A carbon dioxide sensor compositions or carbon dioxide sensor material obtainable according to anyone of claims 6 to 14.

16. A carbon dioxide sensor device comprising at least

    i) a first electrode
    ii) a second electrode spaced from the first electrode
    iii) a carbon dioxide sensor material according to claim 5 or obtainable according to a process according to anyone of claims 6 to 15 between the first electrode and the second electrode
    iv) a system for applying a voltage to the carbon dioxide sensor material, and electric circuitry including at least one measurement system in operative connection with the carbon dioxide sensor material to measure the electrical resistance or impedance relatable to the conductivity of the carbon dioxide sensor material which is dependent on the level of carbon dioxide at the carbon dioxide sensor material as analyte to be detected.

Fig 1:

Glu300

Ade300    Ade320    Ade340    Ade360    Ade380    Ade400

Fig 2:

Fig. 3

**Fig. 4**

$LP_{O2}$-Ade380$_{90}$/Glu300$_{10}$          50 µm

**Fig. 5**                    **Fig. 6**                    **Fig. 7**

$LP_{N2}$-Ade380$_{90}$/Glu300$_{10}$     $LP_{air}$-Ade380$_{90}$/Glu300$_{10}$     $LP_{O2}$-Ade380$_{90}$/Glu300$_{10}$

2 µm                    2 µm                    2 µm

Fig. 8

Fig. 9

**Fig. 10**

O1S                    N1S                    C1S

$LP_{O2}\text{-}Ade380_{90}/Glu300_{10}$

**Fig. 11**

$LP_{air}\text{-}Ade380_{90}/Glu300_{10}$

**Fig. 12**

$LP_{N2}\text{-}Ade380_{90}/Glu300_{10}$

**Fig. 13**

**Fig. 14**

|        | 10%    | 5%    | 1%    | 0.5%  |
|--------|--------|-------|-------|-------|
| ΔR/R   | -14.2% | -6.6% | -2.7% | -1.8% |

**Fig. 15**

**Fig 16**

**Fig 17:**

**Fig. 18:**

**EUROPEAN SEARCH REPORT**

Application Number

EP 22 16 3437

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | JIANG GONG ET AL: "Poly(Ionic Liquid)-Derived Carbon with Site-Specific N-Doping and Biphasic Heterojunction for Enhanced CO2 Capture and Sensing", ANGEWANDTE CHEMIE, WILEY - V C H VERLAG GMBH & CO. KGAA, DE, vol. 129, no. 26, 29 May 2017 (2017-05-29), pages 7665-7671, XP071370881, ISSN: 0044-8249, DOI: 10.1002/ANGE.201702453 * abstract * * figures 2,4 * | 1-5 | INV. G01N33/00 G01N27/02 |
| A,D | OSCHATZ M. ET AL: "A search for selectivity to enable CO 2 capture with porous adsorbents", ENERGY & ENVIRONMENTAL SCIENCE, vol. 11, no. 1, 2018, pages 57-70, XP055947390, Cambridge ISSN: 1754-5692, DOI: 10.1039/C7EE02110K * the whole document * | 1-5 | |
| A | RAMOS-RAMÓN JESÚS A. ET AL: "Nitrogen-Doped Carbon Dots Induced Enhancement in CO2 Sensing Response From ZnO-Porous Silicon Hybrid Structure", FRONTIERS IN CHEMISTRY, vol. 8, 5 May 2020 (2020-05-05), XP055947251, DOI: 10.3389/fchem.2020.00291 * the whole document * | 1-5 | TECHNICAL FIELDS SEARCHED (IPC) G01N |
| A | CN 112 010 281 A (SHANGHAI NAT ENGINEERING RES CENTER NANOTECHNOLOGY CO LTD) 1 December 2020 (2020-12-01) * abstract * * figure 1 * | 1-5 | |

-/--

The present search report has been drawn up for all claims

| Place of search | Date of completion of the search | Examiner |
|---|---|---|
| The Hague | 1 August 2022 | Baranski, Jörg |

EPO FORM 1503 03.82 (P04C01)

Europäisches
Patentamt

European
Patent Office

Office européen
des brevets

# EUROPEAN SEARCH REPORT

Application Number

EP 22 16 3437

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | MARTA SEVILLA ET AL: "N-Doped Polypyrrole-Based Porous Carbons for CO2 Capture", ADVANCED FUNCTIONAL MATERIALS, WILEY - V C H VERLAG GMBH & CO. KGAA, DE, vol. 21, no. 14, 22 July 2011 (2011-07-22), pages 2781-2787, XP001564179, ISSN: 1616-301X, DOI: 10.1002/ADFM.201100291 [retrieved on 2011-05-24] * the whole document * | 1-5 | |
| A,D | WANG HUIZE ET AL: "In Situ Synthesis of Molybdenum Carbide Nanoparticles Incorporated into Laser-Patterned Nitrogen-Doped Carbon for Room Temperature VOC Sensing", ADVANCED FUNCTIONAL MATERIALS, [Online] vol. 31, no. 46, 11 August 2021 (2021-08-11), page 2104061, XP055947427, DE ISSN: 1616-301X, DOI: 10.1002/adfm.202104061 Retrieved from the Internet: URL:https://onlinelibrary.wiley.com/doi/full-xml/10.1002/adfm.202104061> * figure 1 * | 1-5 | TECHNICAL FIELDS SEARCHED (IPC) |

The present search report has been drawn up for all claims

| Place of search | Date of completion of the search | Examiner |
|---|---|---|
| The Hague | 1 August 2022 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

Europäisches
Patentamt
European
Patent Office
Office européen
des brevets

**Application Number**

**EP 22 16 3437**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-5**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

EP 22 16 3437

The Search Division considers that the present European patent application does not comply with the requirements of unity of invention and relates to several inventions or groups of inventions, namely:

```
1. claims: 1-5

    carbon dioxide sensor composition
    1) a first layer comprising a nitrogen-doped carbon material
    having
    a) a carbon content of 75 to 95 wt-%, preferably of 75 to 90
    wt.-% and
    b) a nitrogen content of 2 to 15 wt.-%, preferably 5 to 15
    wt.-% and
    c) an oxygen content of 3 to 12 wt.-%, and
    2) a second layer comprising a nitrogen-doped carbon
    material having a
    a) carbon content of 45 to 74 wt-%, preferably of 50 to 74
    wt.-% and
    b) a nitrogen content of 22 to 45 wt.-%, preferably 23 to 42
    wt.-% and
    c) an oxygen content of 4 to 15 wt.-%
                        ---


2. claims: 6-16

    process for the prepration of carbon dioxide sensor
    compositions,
    a) providing a starting material comprising
    i) at least one precursor material being a material or
    compound that comprises at least one pyrrol or
    imidazole-ring and having a molecular mass of 500 g/mol or
    more and
    ii) at least one film-forming polymer and
    iii) a diluent
    b) coating a substrate with the starting material and
    removing the diluent to obtain a coated substrate
    c) at least partially exposing the coating of the coated
    substrate obtained in step b) to electromagnetic irradiation
    having a wavelength of 780 nm to 1 mm, preferably 1,000 nm
    to 12,500 nm and an intensity and duration sufficient to
    induce at least partial carbonization of the coating
    d) optionally removing the coating prepared in step b) in
    cases where the coated substrate was only partially exposed
    to the electromagnetic irradiation.
                        ---
```

## ANNEX TO THE EUROPEAN SEARCH REPORT
## ON EUROPEAN PATENT APPLICATION NO.

EP 22 16 3437

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2022

| Patent document cited in search report | Publication date | Patent family member(s) | Publication date |
|---|---|---|---|
| CN 112010281 A | 01-12-2020 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020214814 A

- US 20190003998 A

**Non-patent literature cited in the description**

- **S. NAIDU TALAPANENI ; J. HOON LEE ; S. HYUN JE ; O. BUYUKCAKIR ; T. KWON ; K. POLYCHRONOPOULOU ; J. WOOK CHOI ; A. COSKUN ; S. N. TALAPANENI ; J. H. LEE.** *Chemical Blowing Approach for Ultramicroporous Carbon Nitride Frameworks and Their Applications in Gas and Energy Storage,* 2016

- **D. YU ; J. HU ; L. ZHOU ; J. LI ; J. TANG ; C. PENG ; H. LIU.** *Nitrogen-Doped Coal Tar Pitch Based Microporous Carbons with Superior CO2 Capture Performance,* 2018

- **D. WU ; Z. LI ; M. ZHONG ; T. KOWALEWSKI ; K. MATYJASZEWSKI.** *Templated Synthesis of Nitrogen-Enriched Nanoporous Carbon Materials from Porogenic Organic Precursors Prepared by ATRP\*\**

- **J. WEI ; D. ZHOU ; Z. SUN ; Y. DENG ; Y. XIA ; D. ZHAO.** *A Controllable Synthesis of Rich Nitrogen-Doped Ordered Mesoporous Carbon for CO2 Capture and Supercapacitors,* 2013

- **J. GONG ; M. ANTONIETTI ; J. YUAN.** Poly(Ionic Liquid)-Derived Carbon with Site-Specific N-Doping and Biphasic Heterojunction for Enhanced CO2 Capture and Sensing. *Angew. Chemie,* 2017, vol. 129, 7665-7671

- **W. JU ; A. BAGGER ; G.-P. HAO ; A. S. VARELA ; I. SINEV ; V. BON ; B. ROLDAN CUENYA ; S. KASKEL ; J. ROSSMEISL ; P. STRASSER.** Understanding activity and selectivity of metal-nitrogen-doped carbon catalysts for electrochemical reduction of CO. *Nat. Commun.,* 2017, vol. 8, 944

- **M. SEVILLA ; P. VALLE-VIGÓN ; A. B. FUERTES.** N-Doped Polypyrrole-Based Porous Carbons for CO2 Capture. *Adv. Funct. Mater.,* 2011, vol. 21, 2781-2787

- **V. CHANDRA ; S. U. YU ; S. H. KIM ; Y. S. YOON ; D. Y. KIM ; A. H. KWON ; M. MEYYAPPAN ; K. S. KIM.** Highly selective CO2 capture on N-doped carbon produced by chemical activation of polypyrrole functionalized graphene sheets. *Chem. Commun.,* 2012, vol. 48, 735-737

- **S. R. VENNA ; M. A. CARREON.** Highly Permeable Zeolite Imidazolate Framework-8 Membranes for CO2 / CH4 Separation. *J. Am. Chem. Soc.,* 2010, vol. 132, 76-78

- **JOHN W. F. TO et al.** *J. Am. Chem. Soc.,* 2016, vol. 138, 1001-1009

- **Y. ZHAO ; X. LIU ; Y. HAN.** Microporous carbonaceous adsorbents for CO2 separation via selective adsorption. *RSC Adv.,* 2015, vol. 5, 30310-30330

- **Y. ZHAO ; X. LIU ; K. X. YAO ; L. ZHAO ; Y. HAN.** Superior capture of CO2 achieved by introducing extra-framework cations into N-doped microporous carbon. *Chem. Mater.,* 2012, vol. 24, 4725-4734

- **M. OSCHATZ ; M. ANTONIETTI.** A search for selectivity to enable CO2 capture with porous adsorbents. *Energy Environ. Sci.,* 2018, vol. 11, 57-70

- **R. B. K. MAHER ; F. EL-KADY ; VERONICA STRONG ; SERGEY DUBIN.** Laser Scribing of High-Performance and Flexible Graphene-Based Electrochemical Capacitors. *Science,* 2012, vol. 335, 1326-1330

- **J. LIN ; Z. PENG ; Y. LIU ; F. RUIZ-ZEPEDA ; R. YE ; E. L. G. SAMUEL ; M. J. YACAMAN ; B. I. YAKOBSON ; J. M. TOUR.** Laser-induced porous graphene films from commercial polymers. *Nat. Commun.,* 2014, vol. 5, 5-12

- **R. YE ; Y. CHYAN ; J. ZHANG ; Y. LI ; X. HAN ; C. KITTRELL ; J. M. TOUR.** Laser-Induced Graphene Formation on Wood. *Adv. Mater.,* 2017, vol. 29, 1702211

- **V. STRAUSS ; S. DELACROIX ; A. ZIELENIEWSKA ; A. J. FERGUSON ; J. L. BLACKBURN ; S. RONNEBERGER ; F. F. LOEFFLER.** Using carbon laser patterning to produce flexible, metal-free humidity sensors. *ACS Appl. Electron. Mater.,* 2020, vol. 2, 4146-4154

- **H. WANG ; S. DELACROIX ; A. ZIELENIEWSKA ; J. HOU ; N. V. TARAKINA ; D. CRUZ ; I. LAUERMANN ; A. J. FERGUSON ; J. L. BLACKBURN ; V. STRAUSS.** In Situ Synthesis of Molybdenum Carbide Nanoparticles Incorporated into Laser-Patterned Nitrogen-Doped Carbon for Room Temperature VOC Sensing. *Adv. Funct. Mater.,* 2021, 2104061
- **H. WANG ; S. DELACROIX ; O. OSSWALD ; M. ANDERSON ; T. HEIL ; E. LEPRE ; N. LOPEZ-SALAS ; R. B. KANER ; B. SMARSLY ; V. STRAUSS.** Laser-carbonization: Peering into the formation of micro-thermally produced (N-doped)carbons. *Carbon N. Y.,* 2021, vol. 176, 500-510
- **S. DELACROIX ; H. WANG ; T. HEIL ; V. STRAUSS.** Laser-Induced Carbonization of Natural Organic Precursors for Flexible Electronics. *Adv. Electron. Mater.,* 2020, vol. 6, 2000463
- **M. HEPP ; H. WANG ; K. DERR ; S. DELACROIX ; S. RONNEBERGER ; F. LOEFFLER ; B. BUTZ ; V. STRAUSS.** Trained Laser-Patterned Carbon for High-Performance Mechanical Sensors. *Flex. Electron.,* 2021
- **MARCEL SIMSEK ; NONGNOOT WONGKAEW.** Carbon nanomaterial hybrids via laser writing for high-performance non-enzymatic electrochemical sensors: a critical review. *Analytical and Bioanalytical Chemistry,* 2021, vol. 413, 6079-6099
- **D. B. SCHÜPFER ; F. BADACZEWSKI ; J. PEILSTÖCKER ; J. M. GUERRA-CASTRO ; H. SHIM ; S. FIROOZABADI ; A. BEYER ; K. VOLZ ; V. PRESSER ; C. HEILIGER.** Monitoring the thermally induced transition from sp3-hybridized into sp2-hybridized carbons. *Carbon N. Y.,* 2021, vol. 172, 214-227
- **M. PAWLYTA ; J.-N. ROUZAUD ; S. DUBER.** Raman microspectroscopy characterization of carbon blacks: Spectral analysis and structural information. *Carbon N. Y.,* 2015, vol. 84, 479-490